# EUROPEAN PATENT SPECIFICATION

(11) **EP 0 930 915 B1**
(45) Date of publication and mention of the grant of the patent: **31.08.2005**
(21) Application number: 96923351.9
(22) Date of filing: 19.06.1996
(51) Int. Cl.: A61N 2/02

(54) **ELECTROMAGNETIC THERAPEUTIC TREATMENT DEVICE**
APPARATUR ZUR ELEKTROMAGNETISCHEN THERAPIE
DISPOSITIF DE TRAITEMENT ELECTROMAGNETIQUE

(43) Date of publication of application: 28.07.1999
(73) Proprietor: Holcomb, Robert R., Nashville, TN 37203 (US)
(72) Inventor: Holcomb, Robert R., Nashville, TN 37203 (US)
(74) Representative: Fenlon, Christine Lesley
(86) International application number: PCT/US1996/010590
(87) International publication number: WO 1997/000639

(56) References cited:
- WO-A-91/15263

## Description

### BACKGROUND OF THE INVENTION:

### 1. Field Of The Invention:

The present invention relates to magnetic devices for therapeutic application to the human body, particularly for the treatment of various human/animal diseases, complications and disorders such as such as, but not limited to, a) pain and swelling, b) cardiac dysfunction, c) drug resistant seizures and cerebral edema, d) pain and edema sustained in severe bums, e) potentiation of pharmaceutical agents, f) treatment of strokes and protection from cell death following hypoxic injury, g) control of edema and pain and speeding healing following surgical procedures and speeding healing rates of chronic non-healing wounds, and h) control of pain and sludging of sickled cells in sickle cell disease.

### 2. General Background:

Magnetic fields have been applied to the human body for various therapeutic purposes for many centuries. For example, magnetic medical treatment devices for application against selected portions of the human body are disclosed in United States Patent No. 3,921,620; method and apparatus for suppressing neuron action potential firings 5,312,321; magnetic plasters for improving circulation are disclosed in United States Patent No. 4,489,711; magnetic fields for stimulation ofbone growth are disclosed in U.S. Patent No. 4,105,017; and magnetic stimulation of nerve cells has been accomplished with devices such as the Cadwell Magneto-Electric Stimulator (MES-10) manufactured by Cadwell Laboratories, Inc. of Kennewick, Washington.

Various disease states, tissue and organ malfunction may be the result of loss of membrane stability and normal permeability. These membranes may be cellular or intracellular, but in any case represent malfunction of excitable tissue. This malfunction of excitable tissue may be due to alteration of ion channel function. These various disease and states of malfunction may also be related to alteration of receptor sites or agonist sites of enzymes and/or other such dynamic systems within living organisms and more particularly the human animal. A great variety of symptoms and malfunctions may occur, such as, but not limited to, the above listed disease and/or disorder states.

Unfortunately, many types of ailments, including chronic pain, poor localized blood flow, cerebral edema and certain seizures and injuries cannot be successfully treated with conventional drug, physical therapy or surgical therapies. Because such ailments are often untreatable with conventional therapies, there is a need for alternative therapies that relieve these previously untreatable or poorly treatable conditions.

Accordingly, the applicant devised a device that alters the stability of excitable membranes and other charged structures and systems in order to treat aliments of animals. As disclosed in WO 91/15263 the device comprises an electromagnetic device for production of an electromagnetic field for treatment of such disorders, such a device being electromagnetically powered, and having, *inter alia*, an alternating polarity, quadripolar array which generates a 3 dimensional, steep field gradient.

However, it is desirable to improve such a device by increasing the strength and gradient of the 3 dimensional steep gradient field without altering its center charge and symmetry.

### SUMMARY OF THE PRESENT INVENTION:

According to the present invention there is provided a therapeutic electromagnetic treatment device adapted for placement against the bodies of living humans or animals comprising: a plurality of electromagnetic bodies comprising at least four electromagnetic bodies, each having at least one magnetic pole lying substantially in the same plane as a magnetic pole of each of the other electromagnetic bodies; containment means for holding said magnetic poles which lie substantially in the same plane in an orientation in which they define the four vertices of a quadrilateral shape, each of said magnetic poles exerting a magnetic force on the other three poles when said poles are electrically charged; and power supply means for magnetically energizing said electromagnetic bodies such that said energized electromagnetic bodies each generate a magnetic flux field, whereby said magnetic poles which lie substantially in the same plane when energized together form a flux generator head which generates a flux field with a sharp three dimensional gradient; characterised by a ferroconductor flux return ring bolted to the end of a pole which faces away from the animal or human when the device is in use together with a ferromagnetic focusing ring which contains a ferroconductor metal ring and a plurality of flux focusing coils that are attached to the focusing ring and correspond in number and position to the electromagnetic bodies, each of the flux focusing coils having the same polarity as the pole of a corresponding electromagnetic body that defines one of the four vertices of the quadrilateral shape.

### BRIEF DESCRIPTION OF THE DRAWINGS:

Fig. 1 is a schematic diagram of the various components of the electromagnetic treatment device of the invention, including the flux focusing components;
Fig. 2 is a plan view of the four electromagnets of one preferred embodiment of the electromagnetic treatment device of the invention along with the flux focusing components;
Fig. 3 is a perspective view of one of the electromagnets shown in Fig. 2 with the insulation cut away to better see the wire coil;
Fig. 4 is a schematic diagram showing electrical connections between the components of the electromagnetic treatment device of the invention;
Fig. 5 is a perspective view of a preferred embodiment of the electromagnetic treatment device of the invention; and
Fig. 6 is a perspective view of another preferred embodiment of the electromagnetic treatment device of the invention.
Fig. 7 is a diagram of the positive electromagnetic field produced by a device embodying the invention;
Fig. 8 is a schematic diagram showing electrical connections between the components of the electromagnetic treatment device of the invention along with the defibrillation unit;
Fig. 9 is a perspective view of a preferred embodiment of the electromagnetic treatment device of the invention;
Fig. 10 is a perspective view of a preferred embodiment of the electromagnetic treatment device of the invention; and
Fig. 11 is a perspective view of a preferred embodiment of the electromagnetic treatment device of the invention.

### DETAILED DESCRIPTION OF THE PREFERRED EMBODIMENT:

Reference will now be made in detail to the presently preferred embodiments of the invention, examples of which are illustrated in the accompanying drawings. Throughout the drawings like reference characters are used to designate like elements for each of the embodiments.

### A. Pain and Swelling (SMS-P).

### 1. Acute Pain and Edema

A device embodying the present invention can be used to alter nerve cell behavior in a manner that reduces painful sensations by stabilizing excitable membranes and thereby relieving pain, improving blood flow and reducing edema associated with acute injury, inflammation or surgical procedure.

### 2. Chronic Pain

A device embodying the present invention can also be used to alter nerve cell behavior in cases of chronic pain to block the spontaneous pacemaker firing of the chronically malfunctioning pain fiber (mostly Aδ and C-fibers).

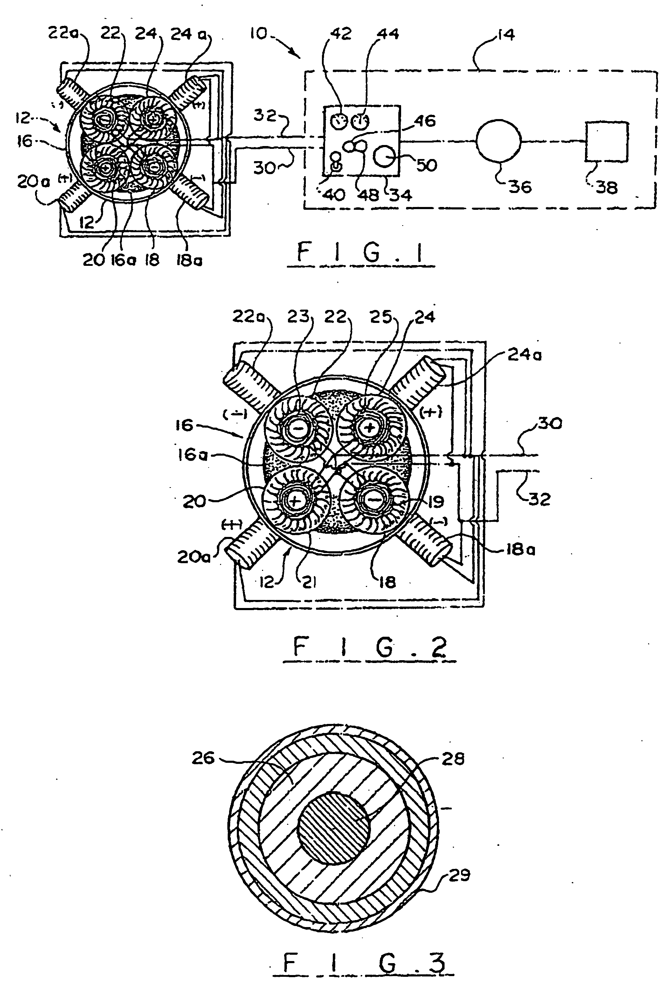

The electromagnetic treatment device of the invention is schematically illustrated in Fig. 1. Treatment device 10 includes a magnetic flux generator 12 and a power source 14. According to the invention, magnetic flux generator 12 comprises a plurality of electromagnetic bodies having at least four magnetic poles substantially in a single plane, the magnetic poles being oriented to define the four vertices of a quadrilateral shape. Preferably, the four magnetic poles comprise two positive and two negative poles, the two positive poles defining opposite diagonal vertices and the two negative poles defining opposite diagonal vertices of the quadrilateral shape. Each of the magnetic poles are magnetically attracted to the two oppositely charged poles and magnetically repelled by the like charged poles. Containment means hold the magnetic poles of the magnetic bodies in the above described configuration. Attached to the containment means are three (3) important and function altering components of the technology. Ferroconductive flux return ring 16a is positioned on the back surface of the described 4 electromagnetic heads, away from the body surface. This ferroconductive ring 16a is designed to return the magnetic flux thereby increasing the strength and gradient without altering the centered charge and symmetry of the 3 dimensional steep gradient magnetic flux field. Thus, this ring enhances the flux field and controls unwanted stray induction currents as well as stray flux from the opposite pole. The four poles of the magnetic flux generator head 12 are provided with a flux focusing ring 16 which surrounds the DC electromagnetic coils on the outer perimeter, stationed midway between the top and bottom of the flux core of the poles, it being about 2.5 inches wide (6.35cm) and ¼ to ½ inches (6.35mm-12.7mm) thick. Attachment means hold the focusing ring in proper location for maximum benefit. Attached to the flux focusing ring are focusing coils 18a, 20a, 22a and 24a. These focusing coils are attached to the flux focusing ring in proximity to a head of like charge and at a 45° to 90° angle to the long axis of the primary flux pole. Power is provided to energize these focusing coils which is controlled through a servo slave mechanism in order to adjust the power in relation to the gradient and energy in the primary core. In this position, the flux focusing coils along with the focusing ring, the flux return ring and the new iron core of this invention make the gradient steeper, increase the field strength and decrease heating and stray high currents. The flux return ring and the flux focusing ring are grounded to reduce stray induction currents and the variables that they add to the therapeutic magnetic field.

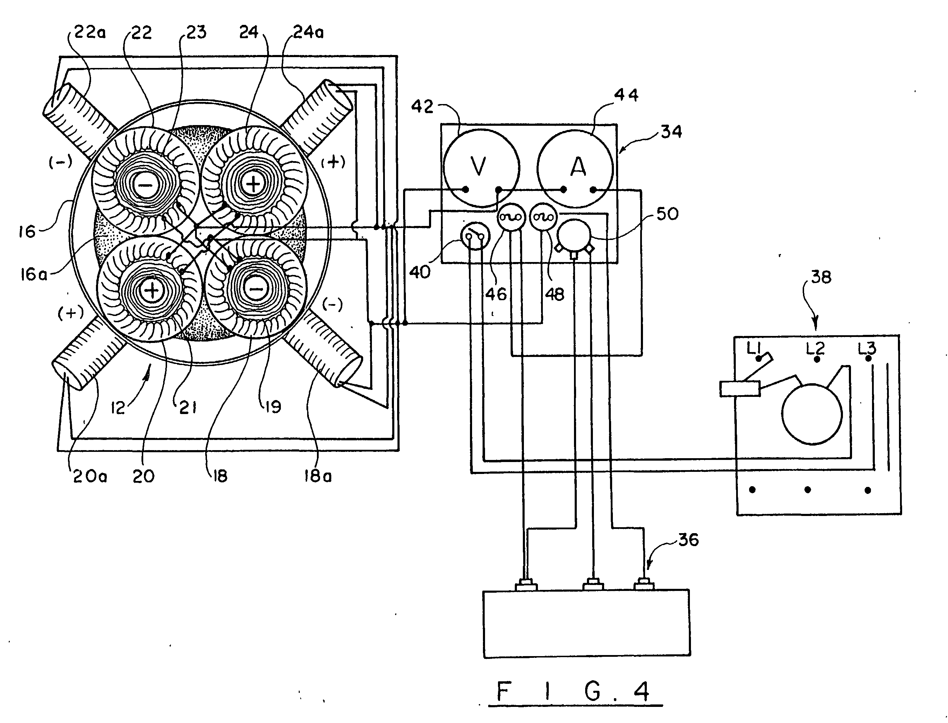

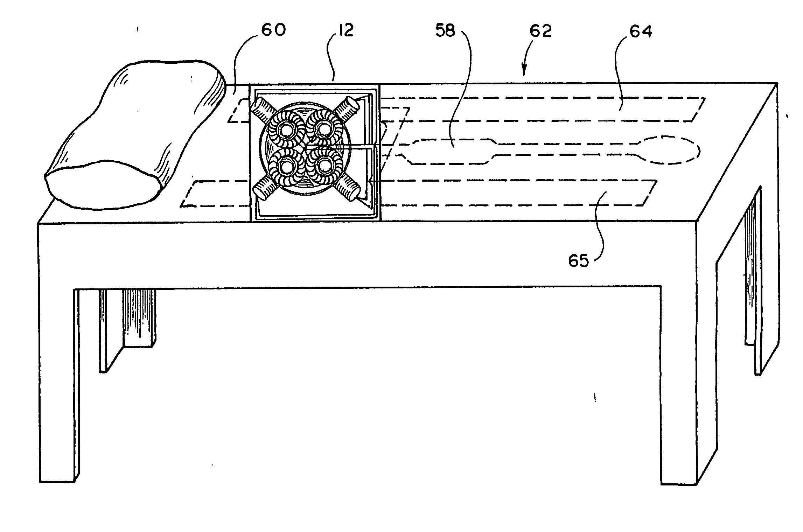

As embodied herein and schematically represented in Figs. 1 and 2, magnetic flux generator 12 comprises four substantially identical electromagnetic bodies 18,20,22 and 24 held on a containment structure. The containment structure may comprise a mounting board, a flux return ring (made of a ferroconductor), a casing or any other structure that will hold electromagnetic bodies 18, 20, 22 and 24 in the desired configuration. In the preferred embodiment, electromagnetic bodies 18 and 22 each form a negative (south) magnetic pole while electromagnetic bodies 20 and 24 each form a positive (north) magnetic pole. The positive and negative magnetic poles of magnetic bodies 18, 20, 22 and 24 are aligned in substantially a single plane and are oriented in a quadrilateral configuration with positive poles oriented diagonally opposite one another and negative poles oriented diagonally opposite one another. Electromagnetic bodies 18, 20, 22 and 24 preferably comprise electromagnetic heads as best shown in Figs. 2 and 3. Each electromagnetic head includes a conducting wire 26 wound around a cast iron core 28. Wire 26 may be comprised of any conducting material, as for example, copper or aluminum. For example, Fig. 3 shows a suitable electromagnet made using a five inch (12.7cm) outer diameter and with a two inch (5.08cm) center core 28 and a one and one half inch (3.81cm) coil space with 3200 turns of # 22 copper wire and covered with insulation 29. As shown in Fig. 2, coils 19,21,23 and 25 of electromagnetic heads 18, 20, 22 and 24, respectively, are each connected to a power source by wires 30 and 32.

The conducting wire 26 is wound around a porous cast iron core 28 in such a fashion as to center the magnetic flux in the geometric center of the iron core. Current flow in an electric conductor emits magnetic flux at right angles to the flow of current. Therefore, the flux is centered in the core. Accordingly, it is preferred that the core be circular.

According to the invention as represented in Fig. 4, power means for magnetically energizing the electromagnetic bodies is provided so that energized electromagnetic bodies can each generate a magnetic flux field. As embodied herein, a power source 14 includes a control unit 34, a direct current generator 36 and an alternating current power source 38. Direct current generator 36 is preferably powered by an alternating current motor, such as a 220 amp AC motor. It is preferred that generator 36 be capable of producing a 30 amp, 120 volt DC current. Control unit 34 includes an on-off power switch 40 for controlling the flow of direct electric current to magnetic flux generator 12. Control unit 34 also includes a volt meter 42 and an amp meter 44 for monitoring of the power and current supplied to magnetic flux generator 12 by direct current generator 36. Fuses 46 and 48 protect magnetic flux generator 12 against power surges. Fuses 46 and 48 may, for example, be 30 amp electric fuses. A rheostat 50 permits regulation of the direct current being supplied to magnetic flux generator 12 at any given time. Rheostat 50 is preferably embodied as any conventional rheostat having a 50 amp, 120 volt capacity. As shown in Figs. 1 and 2, each of the magnetic heads 18, 20, 22 and 24 may be electrically connected with power controller 34 by a single pair of wires 30 and 32. Preferably, each of the magnetic heads 18, 20, 22 and 24 may be individually regulated such that symmetric magnetic power may be balanced among all heads. It is anticipated that each magnetic treatment head could alternatively be individually connected to one of four rheostats in control unit 34 such that electric current supplied to each individual treatment head could be individually regulated. These rheostats could also be held to constant stable magnetic flux by driving the rheostats by a servo-control driven by a computer. The computer would maintain the heads in perfect balance and therefore allow the best possible biological effect, when reduced to practice.

Electromagnetic coils 19 and 23 of electromagnetic heads 18 and 22, and electromagnetic coils 21 and 25 of electromagnetic heads 20 and 24 are preferably connected to the DC generator such that heads 18 and 22 generate magnetic flux fields opposite from the magnetic flux fields generated by heads 20 and 24. As can be seen in Fig. 2, coils 19 and 23 are connected to the DC power source so as to generate a negative magnetic field while coils 21 and 25 are oppositely connected to the DC power source so as to generate a positive magnetic field. In an alternative embodiment of the invention, electromagnetic coils 19, 21, 23 and 25 may be connected to the DC power source such that each head generates a positive magnetic field, a negative magnetic field, or some combination thereof.

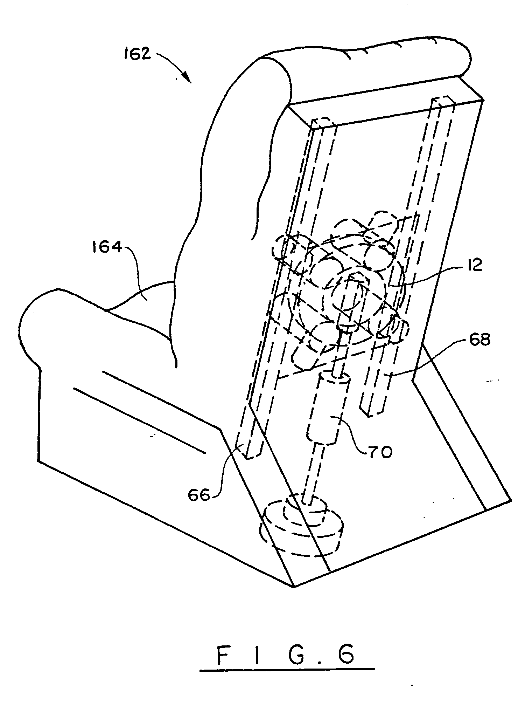

According to the invention, the therapeutic electromagnetic treatment device of the invention may be mounted on a support structure adapted to align the four electromagnets against the body of a living animal. As embodied herein, the support structure may comprise a bench, table, bed, chair or other similar assembly. Fig. 5 shows an elongated treatment table support structure 62 having a magnetic flux generator 12 affixed to the bottom surface thereof. A similar embodiment of the invention is affixed to a hospital intensive care unit bed as an alternative embodiment of the present invention. Flux generator 12 is preferably powered by a power source (not shown) like that described above. Flux generator 12 moves laterally along the bottom surface of table 62 on rails 64 and 65. Flux generator 12 may be moved manually or by a power driven actuating mechanism 58 such that flux generator 12 may be aligned with a desired portion of a human or animal body to which the pulsed or steady state magnetic flux is to be applied. A magnetic flux field is generated by each of the electromagnetic heads on flux generator 12 when the electromagnetic heads are energized. A magnetic flux field so generated by the electromagnetic heads extends above a top planar surface 60 of table 62 such that magnetic flux impinges upon a body on the table surface and exerts very steep three dimensional field gradient upon the organ under treatment.

The therapeutic electromagnetic treatment device of the invention may be otherwise supported by a chair structure, as shown in Fig. 6. Chair 162 includes a seating surface 164 and a vertical back support surface (not shown) and a magnetic flux generator 12. Flux generator 12 moves vertically on rails 66 and 68 in a cavity in the back of chair 162. Flux generator 12 is powered by a power source (not shown) like that described above. The power source may be incorporated in the bottom of chair 162 or may comprise an independent unit. Flux generator 12 may be mounted for manual vertical movement within chair 162 or for automatic movement by a power driven actuating mechanism 70.

The electromagnetic treatment device of this invention has been beneficially applied to the human body to reduce pain, reduce fatigue and improve blood circulation. Several case histories are set forth below.

### CASE 1

A 73 year old female presented with a fourteen year history of severe rheumatoid arthritis of the cervical, thoracic and lumbar spine with progressive pain, stiffness and decreasing mobility. She required assistance to ambulate.

Examination revealed diminished respirations secondary to thoracic radicular pain, decreased range of motion in neck, shoulders and extremities. Both knees were painfully swollen and inflamed.

Medical history revealed she had a total right knee replacement four years prior. She was presently being evaluated for a left total knee replacement. She was on a non-steroidal, anti-inflammatory medication with minimal control of symptoms.

Treatment with local quadripolar permanent magnetic devices as described in United States Patent Application Serial No. 07/171,837 was instituted with excellent pain control reported. Swelling in both knees was reduced and the inflammation disappeared. She continued to take non-steroidal, anti-inflammatory medication concomitantly with the magnetic therapy. She had excellent control of symptoms for 2 ½ years when she experienced an exacerbation of symptoms in the thoracic area with radiculopathy.

Therapy was instituted in her home using the electromagnetic treatment device installed in a recliner chair as shown in Fig. 6. Within a few minutes after treatment had begun, the pain subsided. Within 24 hours of treatment, significant range of motion in the thoracic spine, shoulders and neck returned. Within one week of treatment she had full range of motion in the extremities with sustained improvement.

She had continued to be treated utilizing the electromagnetic device for four months, 3 - 4 times a day, 30 - 45 minutes per treatment. She reports she is pain free, ambulates well without assistance, and has no joint swelling or inflammation.

### CASE 2

A 54 year old female presented with a 10 month history of acute disc herniation with spinal contusion, with surgical intervention, with resultant cord ischemia. She had secondary left thoracic radiculopathy and a sympathetically maintained pain syndrome.

Physical examination revealed scoliosis secondary to muscle spasms. She was receiving pain medication therapy with little relief. She ambulated only with assistance and was unable to carry on normal activities of daily living.

Treatment with the local permanent magnetic devices as described in United States Patent Application Serial No. 07/171,837 was instituted. She reported from 40% - 60% pain reduction for six months. She resumed some routine daily activities. She was evaluated for possible neuro-surgical intervention for remaining pain. A dorsal root entry zone procedure was performed to relieve the radicular and sympathetic pain over the nerve distribution at Thoracic 9, 10, 11 on the left.

The motor tracts were apparently inadvertently overheated during the surgical procedure resulting in plegia of the left lower quadrant including the left lower abdomen, hip, pelvis and leg. She had lost control of bowel and bladder function. After the edema post surgery subsided, she had minimal return of function of left lower quadrant and bowel and bladder function.

She was discharged from the hospital with little hope of recovery. Physical therapy was begun concomitant with treatment with the electromagnetic treatment device installed in a bed as shown in Fig. 5.

She has progressively improved with decrease in pain, increased motor strength and has regained total control of bowel and bladder function.

She has been treated from 1 - 4 hours a day for approximately five months. If treatment of the electromagnetic device is interrupted greater than 48 hours, she has an acute exacerbation of symptoms with increased pain, left sided decreased muscle strength and spasticity of abdominal muscles of hip flexors, dorsal flexors of the foot and less control of bodily functions.

### CASE 3

A 55 year old female presented with a 2 year history of severe Causalgia at Thoracic 9, 10 and 11 with left radicular thoracic somatic and sympathetic pain.

MRI, CT and EMG were negative. She had received multipharmacological therapy with narcotics, non-narcotics pain medication, steroids, non-steroidal, anti-inflammatory drugs and others with little pain relief.

Treatment with the local quadripolar permanent magnetic devices as described in United States Patent Application Serial No. 07/171,837 concomitantly with the electromagnetic treatment device installed in a table was instituted. The devices were worn on a continuous basis. She has received electromagnetic therapy on an average of 4 times a week for 30 minutes to 1 hour for four months.

She reports variable pain reduction post treatment with the electromagnetic device by 50% - 90%. After she has been without electromagnetic therapy for 36 - 48 hours, her pain intensity worsens. She estimates an overall pain reduction at 40%.

### CASE 4

A 67 year old male presented with a 20 year history of Osteoarthritis of the lumbar sacral spine and feet with progressive severe foot and low back pain. Work history revealed that his job required standing on concrete floors from 8 - 10 hours a day. Therapy was instituted in his home using the electromagnetic treatment device installed in a recliner chair as shown in Fig. 6. He was treated once a day at night for 30 minutes to 1 hour. Pain was relieved after the first treatment with sustained relief. He has undergone daily treatments for two months and has reported no exacerbation of pain.

### CASE 5

A 48 year old white male with a history of severe atherosclerosis, coronary artery bypass grafts and stroke presented with painful, cold, blue feet and legs.

Physical examination revealed good pulse but poor small vessel circulation in the lower extremities. The patient was treated in the lower back on the posterior surface with the electromagnetic unit shown in Fig. 5 for 30 minutes. After about 3 minutes, the patient experienced improved color, warming and a sensation of warmth in the feet and legs. This sensation continued to improve with excellent return of color and warmth over the 30 minutes of treatment. These improvements lasted for approximately 4 hours and resulted in longer durations with additional treatments.

### CASE 6

A 63 year old white male presented with a 5 year history of cold feet accompanied by burning paresthesias of the feet. He had been a heavy cigarette smoker. On examination, he had no neurological deficits and had a moderately good dorsalis and posterior pedal pulse. He had marked decrease in small vessel circulation. The patient's lumbosacral area was placed on the electromagnetic device shown in Fig. 5 for 30 minutes. The patient experienced complete reversal of the burning paresthesias and warming of the feet. This improvement was sustained for 3 to 4 hours and the effect was prolonged with repeated treatments.

The beneficial effects of the electromagnetic treatment device of the present invention, at least in part, are brought about by the quadripolar, alternating, center charged, symmetric, static magnetic field impinging upon the cell walls of cells. The steep gradient of these fields brings about a polarization of the lipoprotein matrix of the cell wall such that sodium and calcium channels are blocked in such a fashion as to impede the flux of these ions. Impedance of ion flux blocks the pacemaker effect of damaged or insulted neuronal cell wall membranes (i.e., blocks initiation of a spontaneous depolarization). The control of spontaneous depolarization of neurons brings about: (1) control of pain, and (2) dilatation of peripheral blood vessels by inhibiting excessive outflow of sympathetic nervous discharge to the vessels. This inhibition of sympathetic firing brings about dilatation of the blood vessels. The effect of calcium channel blockade brings about local dilatation blockade brings about local dilatation of vascular smooth muscles and therefore improved blood flow. A portion of the pain relief is likely to be a result of improved blood flow.

### B. Magnetic Treatment Device for Cardiac Dysfunction Static Magnetic Stabilizer - (SMS-C).

A device embodying the present invention is suitable for placement in an intensive care unit bed for the treatment and control of cardiac arrhythmia, improvement of cardiac blood flow and to control chest pain by applying the electromagnetic quadripolar flux generator either over or underneath the chest of a patient with acute myocardial infarction and/or arrhythmia. The three dimensional flux field gradient when applied to the area of the heart controls arrhythmia, improves blood flow, controls angina and protects ischemic myocardial muscle from cell death.

Myocardial infarction with secondary fibrillation and/or heart failure accounts for the leading cause of death in the United States. Myocardial infarction occurs secondary to occlusion of arterial blood supply to a portion of the myocardium. This occlusion can occur either secondary to blockage by atherosclerotic plaques and/or arterial spasm. This event results in chest pain, potential dysarrhythmia, potential fibrillation, and potential heart failure secondary to decrease in inotropic contraction of the myocardium. Each of these events are treated by drug intervention with varying success. In the event that the myocardium fibrillates, this constitutes a medical emergency of the greatest magnitude. The heart must be immediately defibrillated. The current therapy is to place a DC capacitor driven high voltage discharge across the myocardium by applying two paddles to the chest wall and discharging a surge of high intensity DC current. This surge of high voltage DC current will depolarize the myocardium and allow the fastest pacemaker tissue within the heart to assume pacemaker function i.e. the atrial pacemaker. In most instances the myocardium is so irritable and prone to recurrent spontaneous fibrillation that drugs such as I.V. xylocaine must be given to stabilize the electrically irritable myocardium. These drugs are mostly sodium channel blockers and as such they decrease the inotropic and chronotropic contraction of the myocardium. If inadvertent overdosage occurs, the pumping action of the heart may be decreased to below a critical level. Drugs such as epinephrine may need to be used to maintain blood pressure and increase the inotropic and chronotropic contraction of the heart, as well as to increase blood vessel tone. Revival of a patient who fibrillates as a consequence of a myocardial infarction requires rapid manipulation of D/C defibrillation and careful drug manipulation.

The current acute therapy for myocardial infarction and its frequent complications has many side effects. DC defibrillation of the heart causes myocardial damage with each exposure to the D/C discharge. Therefore with each needed defibrillation the chances of recovery progressively decrease. Use of xylocaine to stabilize the myocardium is accompanied by decrease in contraction of the myocardium and decrease in vascular tone. The subsequent use of epinephrine may induce fibrillation and increase the after load on the heart enough to cause congestive heart failure. This condition is clearly a life threatening disorder frequently resulting in death or permanent disability. Because this ailment is often poorly responsive to conventional therapy, there is a need for alternative therapy that will defibrillate the myocardium without cellular damage, stabilize the myocardium without decreasing the desired function, dilate the arteries of the heart for better blood flow, control myocardial pain and prevent cell death.

Accordingly, it is desirable to provide a device that alters myocardial behavior in a manner which stops fibrillation, stabilizes the electrical activity, dilates myocardial arteries and controls chest pain which is secondary to ischemia. It is also desirable to limit the extension of the infarction and to limit cell death which is secondary to ischemia.

A device embodying the present invention alters myocardial behavior in a manner which controls myocardial ischemia, fibrillation, chest pain and extension of the infarction, by altering sodium and calcium channel functions such that the quadripolar, alternating polarity and the subsequent field gradient blocks varying degrees of sodium and calcium channel function. The degree of blockage is related to the gradient and strength of the field. The gradient and the field strength may be manipulated by this technology.

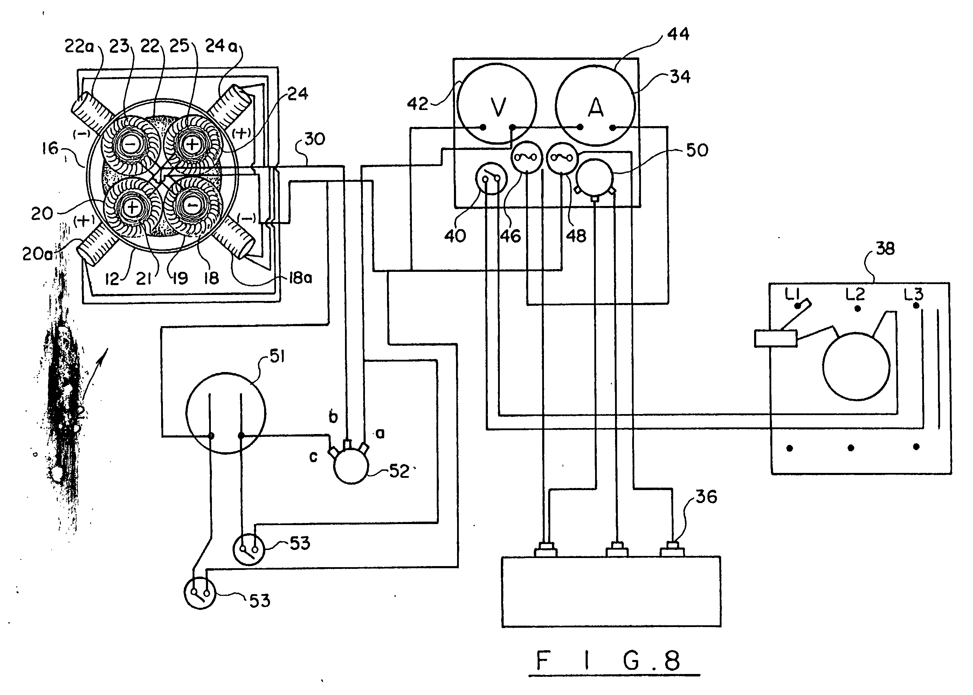

The power supply means 14 used in this embodiment may comprise the arrangement shown in Fig, 8. As shown in Fig. 8, direct current generator 36 is preferably a bridge rectifier and a series of filters. It is preferable that the alternating current to power source 38 be a 120 volt AC source. It is further preferred that capacitor 51 be capable of storing 8000 volts of DC current when switches 53 are closed as in Figure 4. An additional requirement of the invention is that pulse discharge switch 52 capable of closing the discharge circuit through poles b and c when said switch is depressed thereby discharging the capacitor voltage into the flux generator 12, thereby depolarizing the myocardium and defibrillating the heart as a consequence. When said switch 52 is depressed, switch pole a. is open therefore breaking the circuit through a b. It is a further requirement of the invention that when discharge switch 52 is not depressed, circuit through poles a - b is closed.

### C. Magnetic Treatment Device for the Control of Drug Resistant Seizures and Cerebral Edema - Static Magnetic Stabilizer (SMS-E).

This application of the present invention relates to magnetic devices for therapeutic application to the human body, and more particularly to an electromagnetic dual quadripolar treatment device for placement on either side of the human head for control of seizure discharges and cerebral edema.

Seizure activity in the brain is a rather common occurrence. Clinical seizures result from uncontrolled firing of brain neurons. This uncontrolled spontaneous discharge may have projections to many parts of the brain, causing a variety of clinical presentations. These seizures are controlled in many patients by anticonvulsant drugs which work through the alteration of ion channels. The drugs increase the seizure threshold by stabilizing neuron cell wall permeability to ion flux. Many of these seizures are not well controlled by medication nor any other available modalities. A significant number of these seizures are resistant of all modalities of treatment. These patients present to the hospital in a state of continuous uncontrolled seizure activity. This condition is referred to as Status Epilepticus, which is a medical emergency requiring prompt attention. Patients in Status Epilepticus, if not controlled quickly, develop brain hypoxia and resultant hypoxic ischemic encephalopathy. This is a life threatening disorder, frequently resulting in severe brain damage or death. Because this ailment is often untreatable (drug resistant Status Epilepticus) by conventional drugs, there is a need for alternative therapies that stabilize the abnormal irritable neuron cell wall electrophysiology.

Accordingly, it is desirable to provide a device that alters brain neuronal behavior in a manner which stops the abnormal electrical discharge of the neurons, therefore stopping the seizures. Such a device preferably comprises two magnetic flux head generators made with opposite poles facing, having 4 poles per head and alternating polarity such that the field gradient will be enhanced and the total energy applied to the brain will be increased over that which may be delivered by a single head on the device.

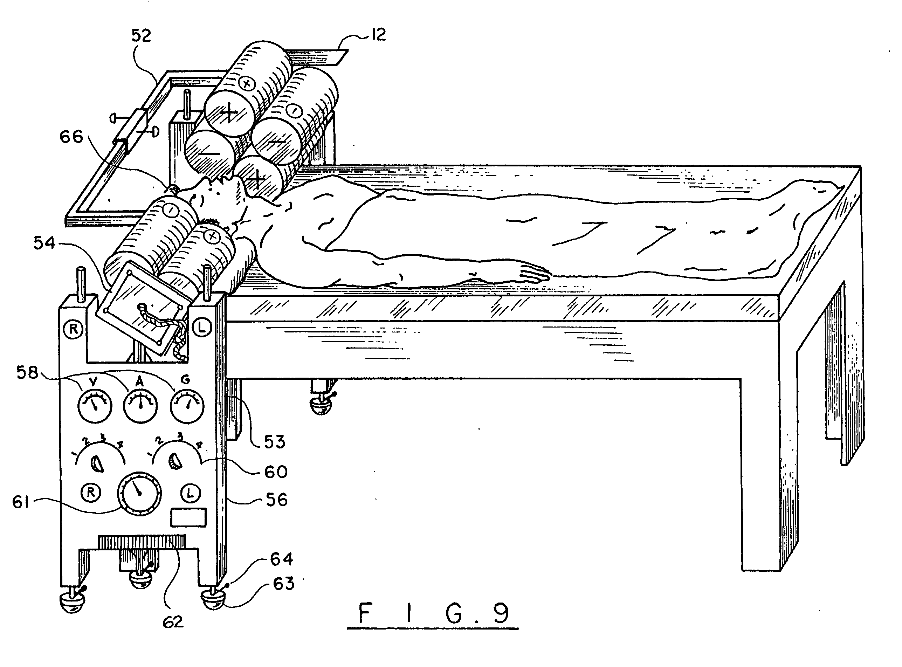

Thus, as shown in Fig. 9, the therapeutic electromagnetic treatment device of the invention preferably comprises two flux generators 12 mounted on a support structure adapted to align the heads such that they are facing each other with the center axis of each electromagnet meeting in the center with opposite poles facing. This configuration increases the peak power of each pole and makes the gradient steeper. The increased peak power and increased slope of the gradient both improve the biological effect. Flux generators 12 are preferably powered by a power source (not shown) like that described above. The flux generators 12 move in the x, y and z axis in order to get appropriate peak magnetic flux and peak magnetic gradient. Flux generators 12 may be moved manually or by a power driven actuating mechanism 54 such that flux generators 12 may be aligned with the desired position of a human or animal head and upper spine to which magnetic flux is to be applied. A magnetic flux field is generated by each of the electromagnetic poles on flux generator 12 when the electromagnetic poles are energized. A magnetic flux field so generated by the electromagnetic heads extends laterally from a planar surface such that the magnetic flux impinges upon an area of the head and/or upper cervical spine of the body positioned between the two flux generators 12. The flux field is managed and manipulated by the flux return and flux focusing ring which hold focusing magnets.

The support means 56 allows both the left and right side sections to be rolled into place forward and locked together by lock bars 52 and 53. Lock bar 53 is underneath the bed. Support means 56 contains the DC power source 14 and provides support for the quadripolar flux generators 12. The support means 56 also contains control meters 58, pole selectors 60 and voltage regulator (DC) 61. A lead counter weight 62 functions to counter balance the flux generators 12. The caster rollers 63 provide the ability to move support means 56 to the bedside.

The patient's head 66 is placed in the static quadripolar field between the two flux generators 12.

The electromagnetic treatment device of Figure 9 has been beneficially applied to the body and head of laboratory animals and humans to control seizures of the brain and to retard and/or reverse brain swelling (cerebral edema).

The beneficial effects of the electromagnetic treatment device of the present invention, at least in part are brought about by the quadripolar, alternating, center charged, symmetric, static magnetic field (maximized by the flux return, flux focusing ring and focusing magnets) impinging upon the cell walls of cells in the brain of animals and man. The steep gradient of these fields brings about a polarization of the lipoprotein matrix of the cell walls such that sodium and calcium channels are blocked in such a fashion as to impede the flux of these ions. Impedance of ion flux blocks the uncontrolled depolarization of brain neurons, thereby preventing the spread of electrical activity which is generated by a seizure focus. Impedance of ion flux also blocks the pacemaker effect of the seizure focus (i.e. blocks initiation of a spontaneous depolarization). The control of spontaneous depolarization of brain neurons brings about control of a seizure focus and control of spread of the erratic depolarization of adjacent neurons. Stabilization of cell walls also inhibits extracellular fluid migration and therefore controls edema of the brain and subsequent cell death.

### Invention Design-Support Data

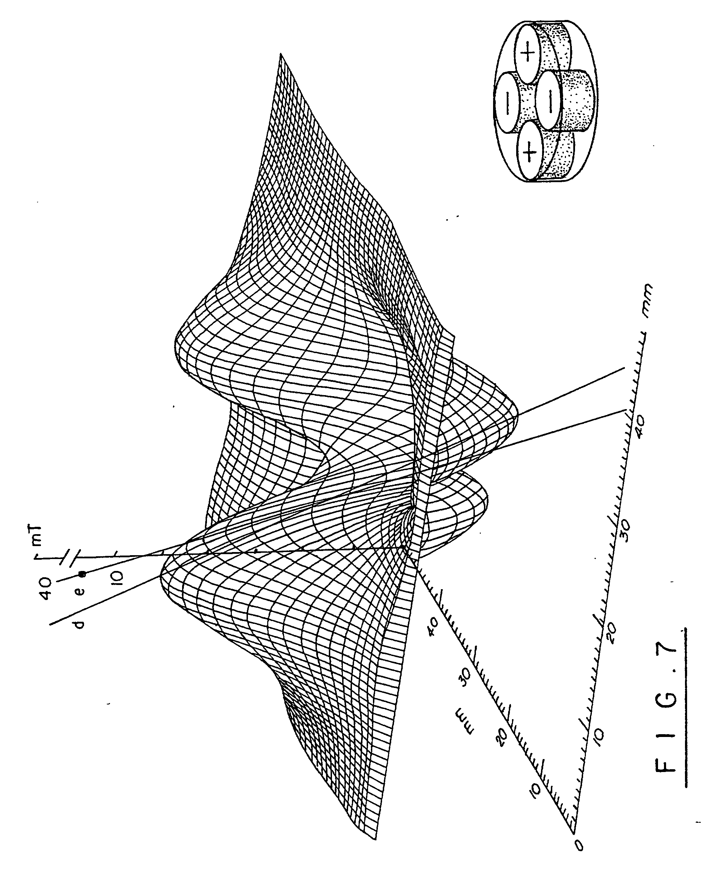

The purpose of this data is to support the design of this invention. The biological effect of this invention is dependent upon the field gradient and the field intensity. The gradient and the intensity may both be increased by approaching one of the flux generator heads of the invention with a second flux generator head with the facing poles being oppositely charged. Fig. 7 demonstrates the change in slope of line d as the peak flux intensity is increased as in sloped line e. The steeper slope of the field gradient e-e over d - d is achieved merely by increasing the magnitude of the peak flux. Field intensity was determined by scanning in a systemic parallel plane 0.3 cm above the surface of the Magna Bloc™ TMNS device with a "Hall Effect" probe and a standard gauss meter; it further demonstrates the increase in the slope of the gradient as the field intensity is increased. The evidence to support the claim of increased energy or peak energy which results from an attracting flux generator is noted in table #1 (See Fig. 4 flux generator 12, Poles A, B, C, D).

**TABLE 1.**

| Reading in Millitesla | A | B | C | D |
|---|---|---|---|---|
| Generator #1 | +73 | -67 | +72 | -72 |
| Generator #1 | +94 | -92 | +87 | -84 |
| + | | | | |
| Generator #2 2.9 inches (7.4cm) from #1 | | | | |
| Generator #1 | +81.4 | -75 | +79 | -80 |
| + | | | | |
| Generator #2 2.9 inches (7.4cm) from #1 | | | | |
| Generator #1 | +78 | -72 | +75 | -75 |
| + | | | | |
| Generator #2 4.2 inches (10.7cm) from #1 | | | | |
| Table #1 is a summation of the flux density in the center of each pole of the flux generator #1: a) In isolation b) when flux generator #2 is faced toward #1 with the poles in an attracting position at varying distances from Generator #1. It is noted the peak flux is increased when a pole is approached by an opposite and attracting pole. As can be noted in Figure 7, this effect of increasing the strength of the field simultaneously increases the gradient. | | | | |

### D. Magnetic Treatment Device for Control of Pain and Edema - Sustained in Severe Burns - Static Magnetic Stabilizer (SMS-B)

A device embodying the present invention may be placed on the human body for control of pain and edema sustained in severe burns.

Severe bums of the human body constitute a medical emergency. Shock secondary to pain is a problem of major magnitude. Shock from hypovolemia is very difficult to control. The burned skin, especially 2nd and 3rd degree burns, leak fluid, electrolytes and protein. These severe consequences of a severe burn are related to stimulation of pain fibers and loss of cell integrity. Current therapy consists of narcotic analgesics, replacement of water, electrolytes and protein, as well as body temperature regulation. These are supportive measures. There is a need for adjunctive therapies that control pain, repair membrane integrity, control edema and stimulate healing.

Accordingly, it is desirable to provide a device that alters pain fiber firing, repairs membrane integrity, controls edema in the area of the burn and promotes healing.

It is also desirable to provide a magnetic device that alters the permeability of burn damaged cells such that they are less permeable to water, ions and protein.

An apparatus for such treatment preferably comprises three movable magnetic flux head generators (with flux and power focusing means, to control the gradient and the power) made with the ability to rotate on each side of the burn bed up to 30° to 45° above the surface of the bed which contains a burn patient.

Alternatively, four magnetic flux head generators may be mounted on a rotating bar, having four poles per head and alternating polarity such that the field gradient will be enhanced and the total energy applied to the burned body will be increased in relationship to the degree of burn.

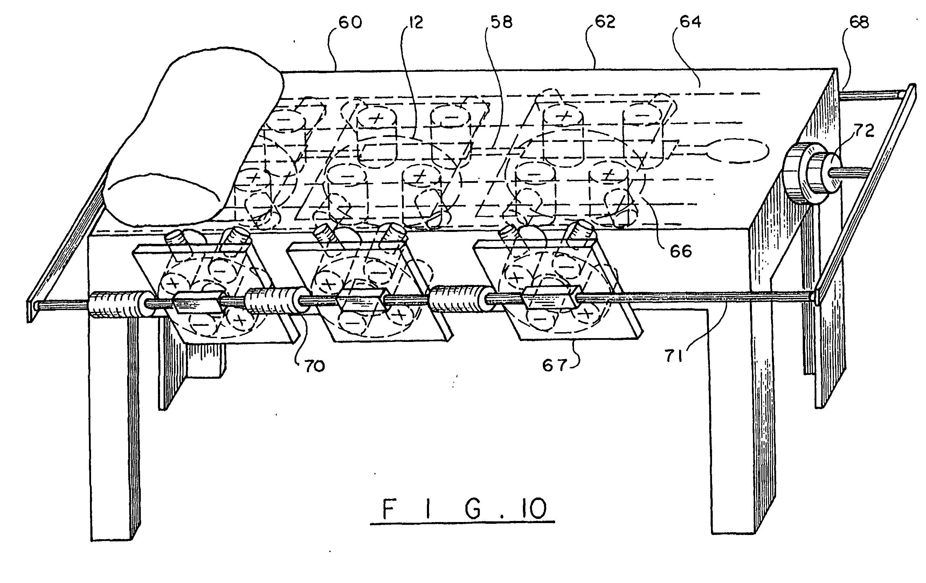

According to the invention, the therapeutic electromagnetic treatment devices of the invention may be mounted on support structures adapted to align the four electromagnets against the body of a living animal which has sustained major burn injury. As embodied herein, the support structure may comprise treatment table, bed or other similar assembly. Fig.10 shows an elongated treatment table support structure 62 having a magnetic flux generator 12 of the invention affixed to the bottom surface and two movable arms thereof. According to the invention, each bank of flux generator heads (12) is constituted of 3 flux generator heads in each bank of generator heads. A similar embodiment of the invention is affixed to a hospital burn unit bed as an alternative embodiment of the present invention. Flux generator 12 is preferably powered by a power source as described in Fig. 4 (not shown) as previously described. Flux generator 12 as contained in the bottom of the bed, moves laterally along the bottom surface of table 62 on rails 64 and 66. Flux generator 12 is moved by a power driven actuating mechanism 58 such that flux generator 12 may be aligned with a desired portion of a human body to which a steady state magnetic flux is to be applied. A magnetic flux field is generated by each of the electromagnetic heads on flux generator 12 when the electromagnetic heads are energized. A magnetic flux field so generated by the electromagnetic heads counter balance support arms (71). Each flux generator head 12 is controlled by a servomotor mechanism 70 which is designed to maintain the focus of the flux field gradient as the counter support arms are rotate up to 30° to 45° above the top planar surface 60 of the table 62 such that magnetic flux impinges upon a body upon the table surface and exerts a very steep three dimensional field gradient upon the burn damaged tissue under treatment. The counter balance support arms 71 are rotated every 10 minutes by servomotor and gear 72. When magnetic flux generator heads 67 or 68 clear the surface 60 of table 62, magnetic flux generator heads 12 in the bottom of the table or bed are turned off by a microswitch in motor and gear 72. When electromagnetic flux generator bank 67 and 68 drop below the surface 60 of table 62, the microswitch turns the electromagnetic flux generator bank in the bottom of the on position such that the electromagnetic flux heads are charged with DC current and produce a static field steep gradient flux field which again impinges upon the body. When magnetic flux generator bank in the bottom of the table or bed is in the off position, the flux generators 67 and 68 are in the on position and are producing the desired magnetic flux field with a steep 3 dimensional gradient.

The therapeutic electromagnetic treatment device of the invention may be otherwise structured without violating the basic mechanism of function.

### E. Magnetic Treatment Device for Potentiation of Pharmaceutical Agents (SMS-D).

A device embodying the present invention may be placed on the human body for control of various disease processes through its interaction with drugs and drug receptors.

Pharmacological agents are used extensively in our society for a variety of diseases, injuries and infectious states. All drugs exert multiple effects on the living organism. The dominant and desired effect is referred to as the therapeutic effect, and the less dominant and undesirable effects are referred to as side effects. All drugs have side effects, many of which are dangerous and/or lethal. Drug effects are mediated by receptor interaction with a receptor agonist. This is an intermediate step in all drug effects.

Accordingly, it is desirable to provide a device that alters receptor configuration and/or drug receptor interaction. The effects may be agonist or antagonist effects. That is, the magnetic field pharmacologic interaction may potentiate or inhibit drug effects and/or side effects.

Such a magnetic flux device preferably comprises an apparatus with four focused magnetic head generators mounted on a flux return ring. These are then surrounded by a flux return focusing ring with a focusing head at a 90° angle to each pole. This device may be focused to any portion of the body, such as the head, in order to bring about potentiation of therapeutic effects of certain drugs.

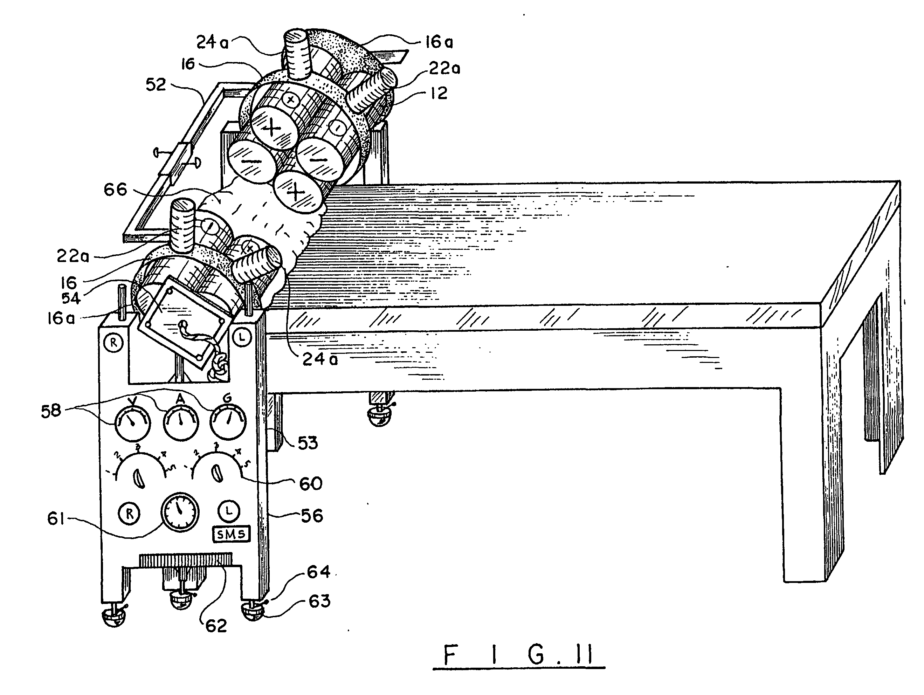

As discussed previously, the therapeutic electromagnetic treatment device of the invention may be mounted on a support structure adapted to align the four electromagnets such that they may be aligned with the other head such that the heads are facing each other with the center axis of each electromagnet meeting in the center with opposite poles facing. This configuration increases the peak power of each pole and makes the gradient steeper. The increased peak power and increased slope of the gradient both improve the biological effect. Fig. 11 shows the means to support the two heads and fasten them together. The two heads are magnetic flux generators. Flux generator 12 is preferably powered by a power source (not shown) like that described above. The flux generators 12 move in the x, y and z axis in order to get appropriate peak magnetic flux and peak magnetic gradient. Flux generators 12 may be moved manually or by a power driven actuating mechanism 54 such that flux generators 12 may be aligned with the desired position of a human or animal head, upper spine or any other portion of the body to which magnetic flux is to be applied. A magnetic flux field is generated by each of the electromagnetic poles on flux generator 12 when the electromagnetic poles are energized. A magnetic flux field so generated by the electromagnetic heads extends laterally from a planar surface such that magnetic flux impinges upon an area of the head and/or upper cervical spine or other parts of the body positioned between the two flux generators 12. The flexible placement allows maximum use for potentiation of pharmaceuticals.

The support means 56 allows both the left and right side sections to be rolled into place forward and locked together by lock bars 52 and 53. Lock bar 53 is underneath the bed. Support means 56 contains the DC power source 14 and provides support for the quadripolar flux generators 12. The support means 56 also contains control meters 58, pole selectors 60 and voltage regulator (DC) 61. A lead counter weight 62 functions to counter balance the flux generators 12. The caster rollers 63 provide the ability to move support means 56 to the bedside.

The patient is placed in the static quadripolar field between the two flux generators 12.

The electromagnetic treatment device of this invention has been beneficially applied to the body and head of laboratory animals and humans to potentiate the therapeutic effect of pharmaceutical agents.

### F. Magnetic Treatment Device for Treatment of Strokes - For Protection from Cell Death Following Hypoxic Injury (SMS-S).

Cerebral ischemia can be either focal or global, implying hypoperfusion of the entire brain. Ischemia is secondary to reduction of cerebral blood flow either locally or globally. Histologically selective neuronal necrosis may occur even after brief periods of global ischemia, and after focal ischemia there may be regions of pan necrosis in the territory of the affected artery. Progressing stroke is an observed deterioration after the initial insult that can occur for 48 to 96 hours. This phenomenon is likely related to a combination of evolving thrombosis and progressive cell death. Cell death occurs secondary to hypoxia, spillage of excitatory neurotransmitters, and spillage of calcium secondary to dying cells in the region.

The electromagnetic treatment device of the invention, for example such as that of Fig. 9, has been beneficially applied to the body and head of laboratory animals to prevent cell death from hypoxia and/or noxious excitatory neurotransmitters.

The magnetic flux field of the invention as described herein may be applied to the human head and/or spinal cord to protect from cell death with evolving strokes or hypoxic ischemic brain insult with associated deficit.

### G. Magnetic Treatment Device of the Invention for Control of Edema and Pain as well as Speeding Healing Rates Following Surgical Procedures and to Increase Healing of Chronic Non-Healing Wounds.

Following surgical procedures, pain, edema, hyperalgesia, healing rate and scar formation are for the large part not treatable except with time and the natural healing process. Non-healing or slowly healing wounds are treated in a variety of ways including hyperbaric oxygen. Non-healing of wounds is mostly due to poor circulation.

A device embodying the present invention may be applied to the human body in the area of a post surgical wound or a chronic non-healing wound. The purpose is to control pain, edema, hyperalgesia, speed healing rates, decrease scar formation and speed the healing of chronic wounds by increasing blood flow to the wound.

The said magnetic flux field, when applied to the area of insult on the animal or human body, will control edema and pain, and speed healing following surgical procedures. It will also increase healing of chronic non-healing wounds.

For example, the patient may be placed in the static quadripolar field between the two flux generators 12 of Fig. 11. Alternately, one head may be used and manipulated such that the area of operative manipulation or poorly healing wound is properly treated by the double or single magnetic flux head of the invention.

### H. Magnetic Treatment Device of this Invention for the Control of Pain and Sludging of Sickled Cells in Sickle Cell Disease (SMS-SC).

Sickle cell anemia is due to an abnormal amino acid sequence in the beta globin chain of adult type (A) hemoglobin. The sickling process is often initiated by low oxygen tension and low PH. The sickled cells obstruct small vessels, and infarcts are frequent. Infarction accounts for the abdominal pain, bone pain, and gradual decrease in the size of the spleen. Current treatment is instituted primarily for the crisis. There is no known effective method for reducing the rate of chronic hemolysis or preventing crisis. The current treatment of choice is red cell transfusion. Other measures are good hydration, oxygen, analgesics and in some cases corticosteroids. A number of antisickling drugs are under evaluation, but none look very promising.

Accordingly, it is desirable to reveal a method of using the variable, quadripolar, three dimensional field gradient of the invention to control the sickling of the blood cells, control pain and sludging associated with sickle cell crisis, and the prophylactic use of the invention in the prevention of sickle cell crisis.

The patient may be placed in the static quadripolar field between the two flux generators 12 of Fig. 11. The position of the heads are controlled by a bedside controller such that they may be located to the area of pain in a patient in sickle cell crisis. The device may also be used prophylactically on a twice daily basis to prevent sickling attacks. Fig. 6 is another embodiment of the technology for sickle cell crisis which involves large areas of the body.

It will be understood that many varying and different embodiments may be made within the scope of the inventive concept herein taught.

## Claims

1. A therapeutic electromagnetic treatment device (10) adapted for placement against the bodies of living humans or animals comprising:
a plurality of electromagnetic bodies comprising at least four electromagnetic bodies (18, 20, 22, 24), each having at least one magnetic pole lying substantially in the same plane as a magnetic pole of each of the other electromagnetic bodies;
containment means for holding said magnetic poles which lie substantially in the same plane in an orientation in which they define the four vertices of a quadrilateral shape, each of said magnetic poles exerting a magnetic force on the other three poles when said poles are electrically charged; and
power supply means (14) for magnetically energizing said electromagnetic bodies (18, 20, 22, 24) such that said energized electromagnetic bodies (18, 20, 22, 24) each generate a magnetic flux field, whereby said magnetic poles which lie substantially in the same plane when energized together form a flux generator head (12) which generates a flux field with a sharp three dimensional gradient;
**characterised by** a ferroconductor flux return ring (16a) bolted to the end of a pole which faces away from the animal or human when the device is in use together with a ferromagnetic focusing ring (16) which contains a ferroconductor metal ring and a plurality of flux focusing coils (18a, 20a,22a, 24a) that are attached to the focusing ring (16) and correspond in number and position to the electromagnetic bodies (18, 20, 22, 24), each of the flux focusing coils (18a, 20a, 22a, 24a) having the same polarity as the pole of a corresponding electromagnetic body (18, 20, 22, 24) that defines one of the four vertices of the quadrilateral shape.

2. The therapeutic electromagnetic treatment device of claim 1, wherein said four magnetic poles defining said quadrilateral shape include two north and two south poles, said two north poles defining opposite diagonal vertices and said two south poles defming opposite diagonal vertices of the quadrilateral shape, each of said magnetic poles being magnetically attracted by said two oppositely charged poles and being magnetically repelled by said like charged pole.

3. The therapeutic electromagnetic treatment device of claim 2, wherein said four electromagnetic bodies (18, 20, 22, 24) comprise four substantially identical electromagnets (18, 20, 22, 24), two of said electromagnets having a north magnetic pole in said substantially single plane and two of said electromagnets having a south magnetic pole in said substantially single plane, each of said four electromagnets (18, 20, 22, 24) generating a magnetic flux field when energized by said power supply means (14).

4. The therapeutic electromagnetic treatment device of any one of claims 1 to 3, wherein said four electromagnets (18, 20, 22, 24) each comprise a cast iron core (28) wound with electrically conducting wire (26).

5. The therapeutic electromagnetic treatment device of claim 4 wherein said electrically conducting wire (26) is copper wire or aluminum wire.

6. The therapeutic electromagnetic treatment device of any preceding claim, wherein said quadrilateral shape is a parallelogram shape.

7. The therapeutic electromagnetic treatment device of claim 6 wherein said parallelogram shape is a rectangle or square shape.

8. The therapeutic electromagnetic treatment device of any preceding claim, wherein said power supply means (14) comprises a direct current generator (36) and is electrically connected to each of said four electromagnetic bodies (18, 20, 22, 24).

9. The therapeutic electromagnetic treatment device of claim 8 further comprising power control means (34) for controlling the amount of electrical power supplied to each of said four electromagnets (18, 20, 22, 24) so as to regulate the magnetic flux field generated by each of said four electromagnets (18, 20, 22, 24).

10. The therapeutic electromagnetic treatment device of any preceding claim, wherein said containment means is mounted on a support structure (62; 162) adapted to align said four electromagnets (18, 20, 22, 24) against the body of a living animal/human.

11. The therapeutic electromagnetic treatment device of claim 10 wherein said support structure (62; 162) comprises an elongated planar table (62) having a first planar surface (60), said first planar surface (60) of said table (62) adapted for supporting the body of a living animal/human against which said four electromagnets (18, 20, 22, 24) are placed, said containment means being movably attached to said planar table (62) for movable alignment with select portions of the living body, the magnetic flux field generated by each of said four electromagnets (18, 20, 22, 24) extending above said first planar surface (60) of said table (62) when said four electromagnets (18, 20, 22, 24) are energized by said power supply means (14).

12. The therapeutic electromagnetic treatment device of claim 11 wherein said planar table (62) has a cavity in which said containment means is movably mounted.

13. The therapeutic electromagnetic treatment device of claim 11 wherein said planar table (62) has a second planar surface opposite said first surface (60), said containment means being movably mounted on said second planar surface.

14. The therapeutic electromagnetic treatment device of claim 10 wherein said support structure (62; 162) comprises a chair (162) having a substantially horizontal seating surface (164) and a substantially vertical back support surface, said seating (164) and back support surfaces adapted for supporting the body of a living animal/human against which said four electromagnets (18, 20, 22, 24) are placed; said containment means being movably attached to said chair (162) for movable alignment with select portions of the living body, the magnetic flux field generated by each of said four electromagnets (18, 20, 22, 24) extending out from the substantially vertical back support surface when said electromagnets (18, 20, 22, 24) are energized by said power supply means (14).

15. The therapeutic electromagnetic treatment device of claim 14 wherein said chair (162) has a vertically extending cavity behind said vertical back support surface and said containment means is mounted for vertical movement within said cavity.

16. The therapeutic electromagnetic treatment device of claim 15 further comprising electrically powered means (70) for moving said containment means vertically and horizontally within said vertically extending cavity.

17. The therapeutic electromagnetic treatment device of any preceding claim, wherein the flux generator head (12) is supported by support means such that the quadripolar flux may be applied to the body area of the myocardium in varying intensity from a steady state to a high voltage pulse capacitor discharge, said discharge being applied to defibrillate the myocardium and to prevent recurrent fibrillation, pain and coronary artery spasms.

18. The therapeutic electromagnetic treatment device of claim 17 wherein said containment means is mounted on a support structure adapted to align said four electromagnets (18, 20, 22, 24) of said flux generator head (12) in the appropriate orientation within the treatment structure such that the myocardium may properly receive the desired flux field, said support structure comprising an attachment means for support of the magnetic flux generator head (12) within an intensive care unit bed within a hospital, the hospital bed is for supporting the body of a living animal/human, the magnetic flux field generated by said four electromagnets (18, 20, 22, 24) of the flux generator head (12) exposes the human or animal to about 200 milli Tesla of energy and steep three dimensional field gradients, when said electromagnets (18, 20, 22, 24) are energized by said power supply means (14).

19. The therapeutic electromagnetic treatment device of any one of claims 1 to 16, wherein two flux generator heads (12) are supported by support means such that the planar surface faces poles of each face in the attracting mode when two flux generator heads (12) are energized.

20. The therapeutic electromagnetic treatment device of claim 19 when appended to claim 3, wherein said containment means is mounted on a support structure adapted to align said four electromagnets (18, 20, 22, 24) of the magnetic flux generator head (12) in the same orientation as the second magnetic flux generator head (12) such that the two heads (12) are facing and in a parallel position.

21. The therapeutic electromagnetic treatment device of claim 20 wherein said support structure comprises a cabinet (56) for the support of each magnetic flux generator head (12), said cabinet (56) is mounted on coasters (63) for easy mobility and has means (52, 53) to fasten the two flux generator heads (12) together once they are in position over the bed or table, the bed is for supporting the body of a living animal/human in which the head and cervical area is placed between the two flux generator heads (12), the magnetic flux field generated by each of said four electromagnets (18, 20, 22, 24) of each flux generator head (12) exposes the human or animal head to 50 up to 500 milli Tesla of energy and steep three dimensional field gradients, when said electromagnets (18, 20, 22, 24) are energized by said power supply means (14).

22. The therapeutic electromagnetic treatment device of any one of claims 1 to 16, wherein the flux generator heads (12) are supported by support means such that the quadripolar flux may be applied to a body area having a severe burn in varying intensity.

23. The therapeutic electromagnetic treatment device of claim 22 wherein said containment means is mounted on a support structure such as a table or bed (62) such that the magnetic flux generator heads (12; 67) are held in the appropriate orientation within the treatment structure such that the burned body may properly receive the desired flux field and wherein said support structure comprises an attachment means for support of the magnetic flux generator heads (12; 67) within a burn unit bed within a hospital, the hospital bed is for supporting the body of a living animal/human, the magnetic flux field generated by said four electromagnets (18, 20, 22, 24) of the flux generator heads (67) exposes the human or animal to about 200 milli Tesla of energy and steep three dimensional field gradients, when said electromagnets (18, 20, 22, 24) are energized by said power supply means (14).

24. The therapeutic electromagnetic treatment device of claim 21 or 23 wherein said power supply means (14) comprises a direct current generator (36) and said power supply means (14) is electrically connected to each of said four electromagnets (18, 20, 22, 24) of the flux generator head (12) along with connection to the flux focusing coils (18a, 20a, 22a, 24a).

25. The therapeutic electromagnetic treatment device of claim 24 wherein said magnetic flux field can be manipulated by the flux return ring (16a) and flux focusing coils (16), when said electromagnets (18, 20, 22, 24) are energized by said power supply means (14).

26. The therapeutic electromagnetic treatment device of claim 24 or 25 further comprising power control means (34) for controlling the amount of electrical power supplied to each of said four electromagnets (18, 20, 22, 24) so as to regulate the magnetic flux field generated by each of said four electromagnets (18, 20, 22, 24) in the or each magnetic flux generator head (12).

27. The therapeutic electromagnetic treatment device of claim 20 wherein said body area of interest is the head or cervical area.

## Patentansprüche

1. Vorrichtung (10) zur elektromagnetischen Therapie, die dafür ausgelegt ist, an den Körper lebender Menschen oder Tiere angelegt zu werden, aufweisend:
eine Anzahl elektromagnetischer Körper, die mindestens vier elektromagnetische Körper (18, 20, 22, 24) umfasst, von denen jeder mindestens einen Magnetpol aufweist, welcher im Wesentlichen in derselben Ebene liegt wie der Magnetpol jedes der anderen elektromagnetischen Körper;
Behältermittel zum Halten der Magnetpole, welche im Wesentlichen in derselben Ebene in einer Ausrichtung liegen, in der sie die vier Eckpunkte einer Vierecksform definieren, wobei jeder der Magnetpole eine magnetische Kraft auf die anderen drei Pole ausübt, wenn die Pole elektrisch geladen sind; und
Leistungszufuhrmittel (14) zum magnetischen Erregen der elektromagnetischen Körper (18, 20, 22, 24), so dass die erregten elektromagnetischen Körper (18, 20, 22, 24) jeweils ein Magnetflussfeld erzeugen, wodurch die Magnetpole, die im Wesentlichen in derselben Ebene liegen, wenn sie erregt sind, zusammen einen Flusserzeugungskopf (12) bilden, welcher ein Flussfeld mit einem scharfen dreidimensionalen Graditen erzeugt;
**gekennzeichnet durch** einen magnetisch leitenden Ring (16a) zur Rückleitung des Flusses, der am Ende eines Poles befestigt ist, welcher vom Tier oder Menschen weg gerichtet ist, wenn die Vorrichtung zusammen mit einem ferromagnetischen Fokussierungsring (16) in Gebrauch ist, welcher Ring einen ferroleitenden Metallring und eine Anzahl an den Fluss fokussierenden Spulen (18a, 20a, 22a, 24a) aufweist, welche am Fokussierungsring (16) angebracht sind und bezüglich der Anzahl und Position dem elektromagnetischen Körper (18, 20, 22, 24) entsprechen, wobei jede der den Fluss fokussierenden Spulen (18a, 20a, 22a, 24a) die gleiche Polarität wie der Pol eines entsprechenden elektromagnetischen Körpers (18, 20, 22, 24) aufweist, welcher einen der vier Eckpunkte der Viereckform definiert.

2. Vorrichtung zur elektromagnetischen Therapie nach Anspruch 1, wobei die vier magnetischen Pole, welche die Viereckform definieren, zwei Nord- und zwei Südpole umfassen, wobei die beiden Nordpole gegenüberliegende diagonale Eckpunkte definieren und die beiden Südpole gegenüberliegende diagonale Eckpunkte der Viereckform definieren, wobei jeder der magnetischen Pole durch die beiden entgegengesetzt aufgeladenen Pole magnetisch angezogen wird und durch den gleich geladenen Pol abgestoßen wird.

3. Vorrichtung zur elektromagnetischen Therapie nach Anspruch 2, wobei die vier elektromagnetischen Körper (18, 20, 22, 24) vier im Wesentlichen identische Elektromagneten (18, 20, 22, 24) umfassen, wobei beide Elektromagneten einen magnetischen Nordpol in der im Wesentlichen einzelnen Ebene aufweisen und zwei der Elektromagneten einen magnetischen Südpol in der im Wesentlichen einzelnen Ebene aufweisen, wobei jeder der vier Elektromagneten (18, 20, 22, 24) ein magnetisches Flussfeld erzeugt, wenn er durch das Leistungsversorgungsmittel (14) erregt wird.

4. Vorrichtung zur elektromagnetischen Therapie nach einem der Ansprüche 1 bis 3, wobei die vier Elektromagneten (18, 20, 22, 24) jeweils einen gegossenen Eisenkern (28) aufweisen, der mit elektrisch leitendem Draht (26) umwickelt ist.

5. Vorrichtung zur elektromagnetischen Therapie nach Anspruch 4, wobei der elektrisch leitende Draht (26) ein Kupferdraht oder Aluminiumdraht ist.

6. Vorrichtung zur elektromagnetischen Therapie nach einem der vorstehenden Ansprüche, wobei die Vierecksform eine Parallelogrammform ist.

7. Vorrichtung zur elektromagnetischen Therapie nach Anspruch 6, wobei die Parallelogrammform ein Rechteck oder eine Quadratform ist.

8. Vorrichtung zur elektromagnetischen Therapie nach einem der vorstehenden Ansprüche, wobei das Leistungsversorgungsmittel (14) einen Direktstromgenerator (36) aufweist und elektrisch mit jedem der vier elektromagnetischen Körper (18, 20, 22, 24) verbunden ist.

9. Vorrichtung zur elektromagnetischen Therapie nach Anspruch 8, weiter aufweisend Leistungssteuerungsmittel (34) zum Steuern der Menge an elektrischer Leistung, die jedem der vier Elektromagneten (18, 20, 22, 24) zugeführt wird, um das magnetische Flussfeld, das durch jeden der vier Elektromagneten (18, 20, 22, 24) erzeugt wird, zu regeln.

10. Vorrichtung zur elektromagnetischen Therapie nach einem der vorstehenden Ansprüche, wobei das Behältermittel auf eine Tragstruktur (62; 162) montiert ist, die dafür ausgelegt ist, die vier Elektromagneten (18, 20, 22, 24) gegen den Körper eines lebenden Tieres/Menschen auszurichten.

11. Vorrichtung zur elektromagnetischen Therapie nach Anspruch 10, wobei die Tragstruktur (62; 162) einen länglichen planaren Tisch (62) aufweist, der eine erste planare Oberfläche (60) hat, wobei die erste planare Oberfläche (60) des Tisches (62) dafür ausgelegt ist, den Körper eines lebenden Tieres/Menschen zu tragen, gegen den die vier Elektromagneten (18, 20, 22, 24) angeordnet sind, wobei das Behältermittel beweglich am planaren Tisch (62) für eine bewegliche Ausrichtung mit ausgewählten Abschnitten des lebenden Körpers angebracht ist, wobei das magnetische Flussfeld, das von jedem der vier Elektromagneten (18, 20, 22, 24) erzeugt wird, sich oberhalb der ersten planaren Oberflächen (60) des Tisches (62) erstreckt, wenn die vier Elektromagneten (18, 20, 22, 24) durch das Leistungszufuhrmittel (14) erregt werden.

12. Vorrichtung zur elektromagnetischen Therapie nach Anspruch 11, wobei der planare Tisch (62) einen Hohlraum aufweist, in dem das Behältermittel beweglich angebracht ist.

13. Vorrichtung zur elektromagnetischen Therapie nach Anspruch 11, wobei der planare Tisch (62) eine zweite planare Oberfläche gegenüber der ersten Oberfläche (60) aufweist, wobei das Behältermittel beweglich auf der zweiten planaren Oberfläche angebracht ist.

14. Vorrichtung zur elektromagnetischen Therapie nach Anspruch 10, wobei die Tragstruktur (62; 162) einen Stuhl (162) aufweist, welcher eine im Wesentlichen horizontale Sitzoberfläche (164) und eine im Wesentlichen vertikale Rückenstützoberfläche aufweist, wobei die Sitz- (164) und Rückenstützoberflächen dafür ausgelegt sind, den Körper eines lebenden Tieres/Menschen, gegen den die vier Elektromagneten (18, 20, 22, 24) gerichtet sind, zu stützen; wobei das Behältermittel beweglich am Stuhl (162) für eine bewegliche Ausrichtung mit ausgewählten Abschnitten des lebenden Körpers angebracht ist, wobei das magnetische Flussfeld, das durch jeden der vier Elektromagneten (18, 20, 22, 24) erzeugt wird, sich von der im Wesentlichen vertikalen Rückenstützoberfläche aus erstreckt, wenn die Elektromagneten (18, 20, 22, 24) durch das Leistungszufuhrmittel (14) erregt werden.

15. Vorrichtung zur elektromagnetischen Therapie nach Anspruch 14, wobei der Stuhl (162) einen sich vertikal erstreckenden Hohlraum hinter der vertikalen Rückenstützoberfläche aufweist und das Behältermittel für eine Vertikalbewegung innerhalb des Hohlraumes angebracht ist.

16. Vorrichtung zur elektromagnetischen Therapie nach Anspruch 15, weiter aufweisend elektrisch betriebene Mittel (70) zum Bewegen der Behältermittel vertikal und horizontal innerhalb des sich vertikal erstreckenden Hohlraumes.

17. Vorrichtung zur elektromagnetischen Therapie nach einem der vorstehenden Ansprüche, wobei der Flussgeneratorkopf (12) durch das Tragmittel getragen wird, so dass der quadropolare Fluss auf dem Körperbereich des Herzmuskels mit variierender Intensität von einem stabilen Zustand zu einer Hochspannungs-Kondensatorpulsentladung angelegt werden kann, wobei die Entladung angelegt wird, um den Herzmuskel zu defibrillieren und um wiederkehrendes Fibrillieren, Schmerzen und um arterielle Herzkrämpfe zu verhindern.

18. Vorrichtung zur elektromagnetischen Therapie nach Anspruch 17, wobei das Behältermittel auf einer Tragstruktur angebracht ist, welche dafür ausgelegt ist, die vier Elektromagneten (18, 20, 22, 24) des Flussgeneratorkopfes (12) in geeigneter Ausrichtung innerhalb der Behandlungsstruktur so auszurichten, dass der Herzmuskel auf geeignete Weise das gewünschte Flussfeld erhalten kann, wobei die Tragstruktur ein Anbringungsmittel zum Stützen des magnetischen Flussgeneratorkopfes (12) innerhalb eines Bettes einer Intensivpflegeeinheit in einem Krankenhaus aufweist, wobei das Krankenhausbett dazu dient, den Körper eines lebenden Tieres/Menschen zu tragen, wobei das magnetische Flussfeld, das durch die vier Elektromagneten (18, 20, 22, 24) des Flussgeneratorkopfes (12) erzeugt wird, den Menschen oder das Tier etwa 200 Millitesla an Energie und steilen dreidimensionalen Feldgradienten aussetzt, wenn die Elektromagneten (18, 20, 22, 24) durch das Leistungsversorgungsmittel (14) erregt werden.

19. Vorrichtung zur elektromagnetischen Therapie nach einem der Ansprüche 1 bis 16, wobei zwei Flussgeneratorköpfe (12) von Tragmitteln gestützt werden, so dass die planaren Oberflächenpole jedes derselben im Anziehungsmodus einander gegenüberstehen, wenn zwei Flussgeneratorköpfe (12) erregt werden.

20. Vorrichtung zur elektromagnetischen Therapie nach Anspruch 19, wenn zusammen mit Anspruch 3 gelesen, wobei das Behältermittel auf einer Tragstruktur angebracht ist, die dafür ausgelegt ist, die vier Elektromagneten (18, 20, 22, 24) des magnetischen Flussgeneratorkopfes (12) in derselben Lage wie den zweiten magnetischen Flussgeneratorkopf (12) auszurichten, so dass die beiden Köpfe (12) einander gegenüberstehen und in einer parallelen Position sind.

21. Vorrichtung zur elektromagnetischen Therapie nach Anspruch 20, wobei die Tragstruktur ein Gehäuse (56) zum Stützen jedes magnetischen Flussgeneratorkopfes (12) aufweist, wobei das Gehäuse (56) auf Untersetzern (63) für einfache Mobilität angebracht ist und Mittel (52, 53) aufweist, um die beiden Flussgeneratorköpfe (12) aneinander zu befestigen, sobald sie über dem Bett oder Tisch in Position sind, wobei das Bett zum Tragen des Körpers eines lebenden Tieres/Menschen gedacht ist, in dem der Kopf und der Himbereich zwischen den beiden Flussgeneratorköpfen (12) angeordnet ist, wobei das magnetische Flussfeld, das von jedem der vier Elektromagneten (18, 20, 22, 24) jedes Flussgeneratorkopfes (12) erzeugt wird, dem menschlichen oder tierischen Kopf von 50 bis 500 Millitesla Energie zuführt, sowie einen steilen dreidimensionalen Feldgradienten, wenn die Elektromagneten (18, 20, 22, 24) durch das Leistungszufuhrmittel (14) erregt werden.

22. Vorrichtung zur elektromagnetischen Therapie nach einem der Ansprüche 1 bis 16, wobei die Flussgeneratorköpfe (12) von Tragmitteln getragen werden, so dass der quadropolare Fluss auf einen Körper aufgebracht werden kann, welcher eine ernsthafte Verbrennung in variierender Intensität aufweist.

23. Vorrichtung zur elektromagnetischen Therapie nach Anspruch 22, wobei das Behältermittel auf einer Tragstruktur wie einem Tisch oder Bett (62) angebracht ist, so dass die den Magnetfluss erzeugenden Köpfe (12; 67) in geeigneter Ausrichtung innerhalb der Behandlungsstruktur gehalten werden, so dass der verbrannte Körper auf geeignete Weise das gewünschte Flussfeld empfangen kann, und wobei die Tragstruktur ein Anbringungsmittel zum Stützen der Generatorköpfe (12; 67) des magnetischen Flusses innerhalb eines Bettes einer Verbrennungseinheit in einem Krankenhaus aufweist, wobei das Krankenhausbett dazu dient, den Körper eines lebenden Tieres/Menschen zu tragen, wobei das magnetische Flussfeld, das von den vier Elektromagneten (18, 20, 22, 24) der Flussgeneratorköpfe (67) getragen wird, den Menschen oder das Tier etwa 200 Millitesla Energie und steilen dreidimensionalen Feldgradienten aussetzt, wenn die Elektromagneten (18, 20, 22, 24) durch das Leistugszufuhrmittel (14) erregt werden.

24. Vorrichtung zur elektromagnetischen Therapie nach Anspruch 21 oder 23, wobei das Leistungszufuhrmittel (14) einen Direktstromgenerator (36) aufweist und das Leistungszufuhrmittel (14) elektrisch mit jedem der vier Elektromagneten (18, 20, 22, 24) des Flussgeneratorkopfes (12) zusammen mit den den Fluss fokusierenden Spulen (18a, 20a, 22a, 24a) verbunden ist.

25. Vorrichtung zur elektromagnetischen Therapie nach Anspruch 24, wobei das magnetische Flussfeld durch den Rückleitungsring (16a) für den Fluss und die den Fluss fokusierenden Spulen (16) beeinflusst werden kann, wenn die Elektromagneten (18, 20, 22, 24) durch das Leistungsversorgungsmittel (14) erregt werden.

26. Vorrichtung zur elektromagnetischen Therapie nach Anspruch 24 oder 25, weiter aufweisend Leistungssteuerungsmittel (34) zum Steuern der Menge an elektrischer Leistung, die jedem der vier Elektromagneten (18, 20, 22, 24) zugeführt wird, um das magnetische Flussfeld zu regulieren, das von jedem der vier Elektromagneten (18, 20, 22, 24) in dem oder jedem magnetischen Flussgeneratorkopf (12) erzeugt wird.

27. Vorrichtung zur elektromagnetischen Therapie nach Anspruch 20, wobei der interessierende Körperbereich der Kopf- oder der Hirnbereich ist.

## Revendications

1. Dispositif de traitement électromagnétique thérapeutique (10) conçu pour être placé sur le corps d'êtres humains ou d'animaux vivants comprenant :
une pluralité de corps électromagnétiques comprenant au moins quatre corps électromagnétiques (18, 20, 22, 24), chacun comportant au moins un pôle magnétique situé sensiblement dans le même plan que les pôles magnétiques de chacun des autres corps électromagnétiques,
un moyen de retenue destiné à retenir lesdits pôles magnétiques qui sont situés sensiblement dans le même plan suivant une orientation dans laquelle ils définissent les quatre sommets d'une forme quadrilatérale, chacun desdits pôles magnétiques exerçant une force magnétique sur les trois autres pôles lorsque lesdits pôles sont chargés électriquement, et
un moyen d'alimentation électrique (14) destiné à exciter magnétiquement lesdits corps électromagnétiques (18, 20, 22, 24) de sorte que lesdits corps électromagnétiques excités (18, 20, 22, 24) génèrent chacun un champ de flux magnétique, grâce à quoi lesdits pôles magnétiques, qui sont situés sensiblement dans le même plan, lorsqu'ils sont excités ensemble, forment une tête de générateur de flux (12) qui génère un champ de flux présentant un gradient à trois dimensions accentué,
**caractérisé par** une bague de retour de flux ferroconductrice (16a) boulonnée à l'extrémité d'un pôle qui est détourné de l'animal ou de l'être humain lorsque le dispositif est en utilisation en même temps qu'une bague de focalisation ferromagnétique (16) qui contient une bague de métal ferroconducteur et une pluralité de bobines de focalisation de flux (18a, 20a, 22a, 24a) qui sont fixées à la bague de focalisation (16) et correspondent, en nombre et en position, aux corps électromagnétiques (18, 20, 22, 24), chacune des bobines de focalisation de flux (18a, 20a, 22a, 24a) présentant la même polarité que le pôle d'un corps électromagnétique correspondant (18, 20, 22, 24) qui définit l'un des quatre sommets de la forme quadrilatérale.

2. Dispositif de traitement électromagnétique thérapeutique selon la revendication 1, dans lequel lesdits quatre pôles magnétiques définissant ladite forme quadrilatérale, comprennent deux pôles nord et deux pôles sud, lesdits deux pôles nord définissant des sommets opposés selon la diagonale, et lesdits deux pôles sud définissant des sommets opposés selon la diagonale de la forme quadrilatérale, chacun desdits pôles magnétiques étant attiré magnétiquement par lesdits deux pôles chargés de manière opposée, et étant magnétiquement repoussés par ledit pôle chargé de manière identique.

3. Dispositif de traitement électromagnétique thérapeutique selon la revendication 2, dans lequel lesdits quatre corps électromagnétiques (18, 20, 22, 24) comprennent quatre électroaimants sensiblement identiques (18, 20, 22, 24), deux desdits électroaimants présentant un pôle magnétique nord dans ledit plan sensiblement unique et deux desdits électroaimants présentant un pôle magnétique sud dans ledit plan sensiblement unique, chacun desdits quatre électroaimants (18, 20, 22, 24) générant un champ de flux magnétique lorsqu'il est excité par ledit moyen d'alimentation électrique (14).

4. Dispositif de traitement électromagnétique thérapeutique selon l'une quelconque des revendications 1 à 3, dans lequel lesdits quatre électroaimants (18, 20, 22, 24) comprennent chacun un noyau en fonte (28), un fil électriquement conducteur (26) étant enroulé autour de celui-ci.

5. Dispositif de traitement électromagnétique thérapeutique selon la revendication 4, dans lequel ledit fil électriquement conducteur (26) est un fil de cuivre ou un fil d'aluminium.

6. Dispositif de traitement électromagnétique thérapeutique selon l'une quelconque des revendications précédentes, dans lequel ladite forme quadrilatérale est une forme de parallélogramme.

7. Dispositif de traitement électromagnétique thérapeutique selon la revendication 6, dans lequel ladite forme de parallélogramme est une forme rectangulaire ou carrée.

8. Dispositif de traitement électromagnétique thérapeutique selon l'une quelconque des revendications précédentes, dans lequel ledit moyen d'alimentation électrique (14) comprend un générateur de courant continu (36) et est électriquement connecté à chacun desdits quatre corps électromagnétiques (18, 20, 22, 24).

9. Dispositif de traitement électromagnétique thérapeutique selon la revendication 8, comprenant en outre un moyen d'alimentation électrique (34) destiné à commander la quantité de courant électrique fournie à chacun des quatre électroaimants (18, 20, 22, 24) de manière à réguler le champ de flux magnétique généré par chacun desdits quatre électroaimants (18, 20, 22, 24).

10. Dispositif de traitement électromagnétique thérapeutique selon l'une quelconque des revendications précédentes, dans lequel ledit moyen de retenue est monté sur une structure de support (62 ; 162) conçue pour aligner lesdits quatre électroaimants (18, 20, 22, 24) sur le corps d'un animal ou d'un être humain vivant.

11. Dispositif de traitement électromagnétique thérapeutique selon la revendication 10, dans lequel ladite structure de support (62 ; 162) comprend une table plane allongée (62) présentant une première surface plane (60), ladite première surface plane (60) de ladite table (62) étant conçue pour supporter le corps d'un animal ou d'un être humain vivant sur lequel lesdits quatre électroaimants (18, 20, 22, 24) sont placés, ledit moyen de retenue étant fixé de manière mobile à ladite table plane (62) en vue d'un alignement mobile avec lesdites parties sélectionnées du corps vivant, le champ de flux magnétique généré par chacun desdits quatre électroaimants (18, 20, 22, 24) s'étendant au-dessus de ladite première surface plane (60) de ladite table (62) lorsque lesdits quatre électroaimants (18, 20, 22, 24) sont excités par ledit moyen d'alimentation électrique (14).

12. Dispositif de traitement électromagnétique thérapeutique selon la revendication 11, dans lequel ladite table plane (62) comporte une cavité dans laquelle ledit moyen de retenue est monté de manière mobile.

13. Dispositif de traitement électromagnétique thérapeutique selon la revendication 11, dans lequel ladite table plane (62) présente une seconde surface plane opposée à ladite première surface (60), ledit moyen de retenue étant monté de manière mobile sur ladite seconde surface plane.

14. Dispositif de traitement électromagnétique thérapeutique selon la revendication 10, dans lequel ladite structure de support (62 ; 162) comprend une chaise (162) présentant une surface d'assise sensiblement horizontale (164) et une surface de support de dossier sensiblement verticale, lesdites surfaces de support d'assise (164) et de dossier étant conçues pour supporter le corps d'un animal ou d'un être humain vivant sur lequel lesdits quatre électroaimants (18, 20, 22, 24) sont placés, ledit moyen de retenue étant fixé de manière mobile à ladite chaise (162) en vue d'un alignement mobile avec les parties sélectionnées du corps humain, le champ de flux magnétique généré par chacun desdits quatre électroaimants (18, 20, 22, 24) s'étendant à l'extérieur depuis la surface de support de dossier sensiblement verticale, lorsque lesdits électroaimants (18, 20, 22, 24) sont excités par ledit moyen d'alimentation électrique (14).

15. Dispositif de traitement électromagnétique thérapeutique selon la revendication 14, dans lequel ladite chaise (162) comporte une cavité s'étendant verticalement derrière ladite surface de support de dossier verticale et ledit moyen de retenue est monté en vue d'un mouvement vertical à l'intérieur de ladite cavité.

16. Dispositif de traitement électromagnétique thérapeutique selon la revendication 15, comprenant en outre un moyen alimenté électriquement (70) destiné à déplacer ledit moyen de retenue verticalement et horizontalement à l'intérieur de ladite cavité s'étendant verticalement.

17. Dispositif de traitement électromagnétique thérapeutique selon l'une quelconque des revendications précédentes, dans lequel la tête de générateur de flux (12) est supportée par un moyen de support de sorte que le flux quadripolaire puisse être appliqué à la zone du corps du myocarde en faisant varier l'intensité depuis un état stable à une décharge capacitive par impulsion à haute tension, ladite décharge étant appliquée pour défibriller le myocarde et pour empêcher une fibrillation, une douleur et des spasmes artériels coronariens récurrents.

18. Dispositif de traitement électromagnétique thérapeutique selon la revendication 17, dans lequel ledit moyen de retenue est monté sur une structure de support conçue pour aligner lesdits quatre électroaimants (18, 20, 22, 24) de ladite tête de générateur de flux (12) selon l'orientation appropriée à l'intérieur de la structure de traitement, de sorte que le myocarde puisse recevoir correctement le champ de flux souhaité, ladite structure de support comprenant un moyen de fixation pour le support de la tête du générateur de flux magnétique (12) à l'intérieur d'un lit d'unité de soins intensifs dans un hôpital, le lit d'hôpital est destiné à supporter le corps d'un animal ou d'un être humain vivant, le champ de flux magnétique généré par lesdits quatre électroaimants (18, 20, 22, 24) de la tête de générateur de flux (12) expose l'être humain ou l'animal à environ 200 milliteslas d'énergie et à des gradients de champ accentués en trois dimensions, lorsque lesdits électroaimants (18, 20, 22, 24) sont excités par ledit moyen d'alimentation électrique (14).

19. Dispositif de traitement électromagnétique thérapeutique selon l'une quelconque des revendications 1 à 16, dans lequel deux têtes de générateur de flux (12) sont supportées par un moyen de support tel que la surface plane est orientée vers les pôles de chaque face dans le mode d'attraction lorsque les deux têtes de générateur de flux (12) sont excitées.

20. Dispositif de traitement électromagnétique thérapeutique selon la revendication 19, lorsqu'elle dépend de la revendication 3, dans lequel ledit moyen de retenue est monté sur une structure de support conçue pour aligner lesdits quatre électroaimants (18, 20, 22, 24) de la tête de générateur de flux magnétique (12) selon la même orientation que la seconde tête de générateur de flux magnétique (12) de sorte que les deux têtes (12) soient face à face et en position parallèle.

21. Dispositif de traitement électromagnétique thérapeutique selon la revendication 20, dans lequel ladite structure de support comprend une armoire (56) pour le support de chaque tête de générateur de flux magnétique (12), ladite armoire (56) est montée sur des patins (63) en vue d'une mobilité aisée et comporte un moyen (52, 53) pour attacher les deux têtes de générateur de flux (12) ensemble une fois qu'elles sont en position au-dessus du lit ou de la table, le lit est destiné à supporter le corps d'un animal ou d'un être humain vivant dont la tête et la zone cervicale sont placées entre les deux têtes de générateur de flux (12), le champ de flux magnétique généré par chacun desdits quatre électroaimants (18, 20, 22, 24) de chaque tête de générateur de flux (12) expose la tête de l'être humain ou l'animal à 50 jusqu'à 500 milliteslas d'énergie, et à des gradients de champ accentués en trois dimensions, lorsque lesdits électroaimants (18, 20, 22, 24) sont excités par ledit moyen d'alimentation électrique (14).

22. Dispositif de traitement électromagnétique thérapeutique selon l'une quelconque des revendications 1 à 16, dans lequel les têtes de générateur de flux (12) sont supportées par un moyen de support tel que le flux quadripolaire puisse être appliqué à une zone du corps présentant une brûlure sévère avec une intensité variable.

23. Dispositif de traitement électromagnétique thérapeutique selon la revendication 22, dans lequel ledit moyen de retenue est monté sur une structure de support telle qu'une table ou un lit (62) de sorte que les têtes de générateur de flux magnétique (12 ; 67) soient conservées suivant l'orientation appropriée à l'intérieur de la structure de traitement, de sorte que le corps brûlé puisse recevoir correctement le champ de flux souhaité, et dans lequel ladite structure de support comprend un moyen de fixation pour le support des têtes de générateur de flux magnétique (12 ; 67) dans un lit d'unité de brûlés dans un hôpital, le lit d'hôpital est destiné à supporter le corps d'un animal ou d'un être humain vivant, le champ de flux magnétique généré par lesdits quatre électroaimants (18, 20, 22, 24) des têtes de générateur de flux (67) expose l'être humain ou l'animal à environ 200 milliteslas d'énergie et à des gradients de champ accentués en trois dimensions, lorsque lesdits électroaimants (18, 20, 22, 24) sont excités par ledit moyen d'alimentation électrique (14).

24. Dispositif de traitement électromagnétique thérapeutique selon la revendication 21 ou 23, dans lequel ledit moyen d'alimentation électrique (14) comprend un générateur de courant continu (36) et ledit moyen d'alimentation électrique (14) est électriquement connecté à chacun desdits quatre électroaimants (18, 20, 22, 24) de la tête de générateur de flux (12) en même temps qu'une connexion aux bobines de focalisation de flux (18a, 20a, 22a, 24a).

25. Dispositif de traitement électromagnétique thérapeutique selon la revendication 24, dans lequel ledit champ de flux magnétique peut être manipulé par la bague de retour de flux (16a) et les bobines de focalisation de flux (16), lorsque lesdits électroaimants (18, 20, 22, 24) sont excités par ledit moyen d'alimentation électrique (14).

26. Dispositif de traitement électromagnétique thérapeutique selon la revendication 24 ou 25 comprenant en outre un moyen de commande d'alimentation électrique (34) destiné à commander la quantité de courant électrique fournie à chacun desdits quatre électroaimants (18, 20, 22, 24) de manière à réguler le champ de flux magnétique généré par chacun desdits quatre électroaimants (18, 20, 22, 24) dans la tête de générateur de flux magnétique (12) ou bien dans chacune de celles-ci.

27. Dispositif de traitement électromagnétique thérapeutique selon la revendication 20, dans lequel ladite zone du corps concernée est la tête ou la zone cervicale.
